(19) Europäisches Patentamt
European Patent Office
Office européen des brevets

(11) Publication number: 0 219 990 A1

(12) # EUROPEAN PATENT APPLICATION

(21) Application number: 86307432.4

(51) Int. Cl.[4]: C 07 D 261/10

(22) Date of filing: 26.09.86

(30) Priority: 26.09.85 HU 368485

(43) Date of publication of application: 29.04.87 Bulletin 87/18

(84) Designated Contracting States: AT BE DE FR NL SE

(71) Applicant: BIOGAL GYOGYSZERGYAR
Pallagi u. 13.
H-4042 Debrecen(HU)

(72) Inventor: Lempert, Karoly
1073 Budapest
Kertesz u. 50(HU)

(72) Inventor: Doleschall, Gabor
1122 Budapest
Hajnoczy u. 4(HU)

(72) Inventor: Fetter, Jozsef
1119 Budapest
Hengermalom u. 8/b(HU)

(72) Inventor: Hornyak, Gyula
1111 Budapest
Bercsenyi u. 10(HU)

(72) Inventor: Nyitrai, Jozsef
1172 Budapest
Nagyszekes u. 10(HU)

(72) Inventor: Simig, Gyula
1126 Budapest
Hollosy S.u. 254(HU)

(72) Inventor: Gombos, Zsuzsanna
1145 Budapest
Bosnyak u. 1/a(HU)

(72) Inventor: Nagy, Jozsef
1112 Budapest
Torokbalinti u. 46/b(HU)

(72) Inventor: Zauer, Karoly
2000 Szentendre
Bukkos part 43(HU)

(72) Inventor: Elek, Sandor
4032 Debrecen
Nemet L. u. 25(HU)

(72) Inventor: Mihok, Miklos
4032 Debrecen
Szabo I. altabornagy ter 2(HU)

(72) Inventor: Mihok, Ildiko
4032 Debrecen
Szabo I. altabornagy ter 2(HU)

(74) Representative: Tubby, David George et al,
MARKS & CLERK 57-60 Lincoln's Inn Fields
London WC2A 3LS(GB)

(54) **Process for the preparation of 3-chloro-5-isoxazoleacetic acid.**

(57) 3-Chloro-5-isoxazoleacetic acid is prepared from 3-chloro-5-isoxazolepropionic acid by conversion of the latter to E-3-chloro-5-isoxazoleacrylic acid. This is first converted to the acid chloride, then to the acid azide, which is subjected to a Curtius degradation and hydrolysed and the hydrolysed product is condensed with hydroxylamine to give the corresponding nitrile, which is hydrolysed to give said 3-chloro-5-isoxazoleacetic acid.

EP 0 219 990 A1

Croydon Printing Company Ltd.

# PROCESS FOR THE PREPARATION OF 3-CHLORO-5-ISOXAZOLEACETIC ACID

The present invention relates to a novel process for the preparation of 3-chloro-5-isoxazoleacetic acid of the formula (I):

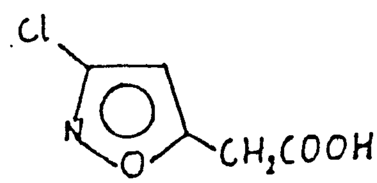

(I)

This compound can be used to form a side chain in the 7-position of β-lactam molecules as is described in GB 1 464 377 and in Hungarian patent application No. 2725/85.

Several experiments have been carried out for preparing the compound of formula (I). In GB 1 464 377, 3-chloro-5-methylisoxazole is used as the starting material. However, extreme temperature conditions (-85°C) are required to give a yield of only 46%.

In the process of preparing the compound of formula (I) described by P. Lugosi et al [Tetrahedron 37,3061 (1981)], 3-chloro-5-isoxazolepropionic acid of formula (II):

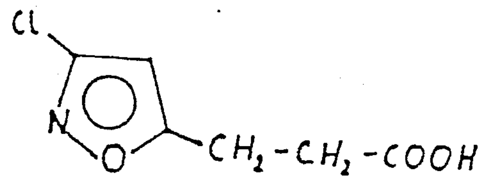

(II)

is used as the starting material; the yield of the compound of formula (I) is, however, similarly low.

In its broadest aspect, the present invention consists a process for preparing 3-chloro-5-isoxazoleacetic acid by dehydrating 3-chloro-5-isoxazoleacetaldehyde oxime to 3-chloro-5-cyanomethylisoxazole, which is then hydrolyzed to said 3-chloro-5-isoxazoleacetic acid.

In the course of our investigations to find a more economic preparation method for the compound of formula (I), we have found that the compound of formula (II) can be employed as the starting material to obtain E-3-chloro-5-isoxazoleacrylic acid of formula (III):

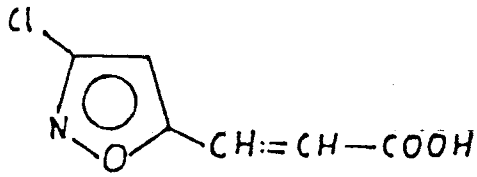

(III)

as an intermediate for preparing the desired compound of formula (I).

Two closely related methods were developed by us for the preparation of the compound of the formula (III).

a) one of these processes comprises activating the carboxylic group of 3-chloro-5-isoxazolepropionic acid [formula (II)], suitably by forming the acid chloride of formula (IV):

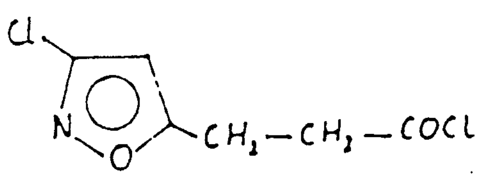

(IV)

This acid chloride is reacted with a halogenating agent, preferably with sulfuryl chloride or bromine, and the resulting compound, halogenated in its side chain, is treated with a strong base and then with an acid to give E-3-chloro-5-isoxazoleacrylic acid of formula (III).

b) The other process comprises activating 3-chloro-5-isoxazolepropionic acid [formula (II)] in the form of an ester of formula (V):

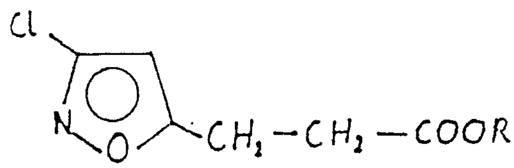

(V)

wherein R represents a $C_1$-$C_6$ alkyl group, treating the compound of formula (V) with a strong base, preferably with a sodium alkoxide, sodium hydride, sodium amide or potassium t-butoxide, reacting the thus-prepared β-ketoester of formula (VI):

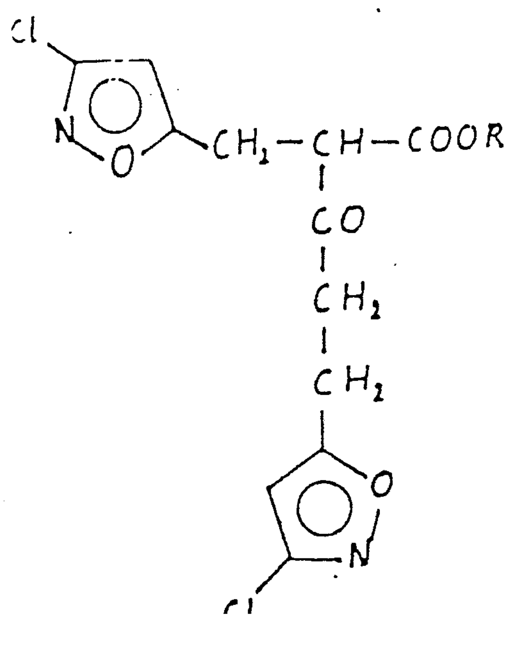

(VI)

wherein R is as defined above, with a halogenating agent, e.g. bromine or chlorine (preferably about two moles thereof), and converting without isolation the resulting halogenated product to E-3-chloro-5-isoxazoleacrylic acid of formula (III) by treating it with a base or an acid.

In the following steps of the process of the invention, the compound of formula (III) prepared by either of the above two processes is used as a starting material which is converted in the first step to an activated species, preferably to the acid chloride. This latter compound is reacted with an azide, e.g. sodium azide, to give the acyl azide of formula (VII):

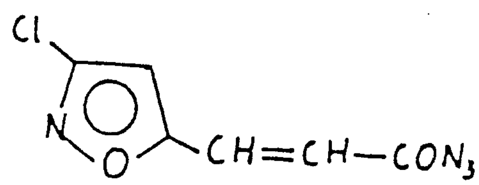

(VII)

which is subjected to a Curtius degradation and then hydrolyzed by an acid. The resulting product is reacted with hydroxylamine to give the oxime of formula (VIII):

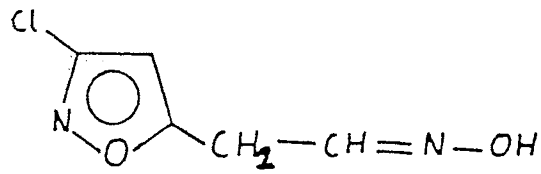

(VIII)

which is treated with a dehydrating agent, preferably with thionyl chloride, to obtain the nitrile of formula (IX):

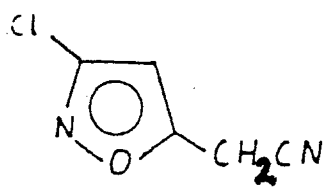

(IX)

which is then hydrolyzed, without isolation, preferably in a strongly acidic medium to the desired 3-chloro-5-isoxazoleacetic acid of formula (I).

Preferably, the activated 3-chloro-5-isoxazolepropionic acid or said β-ketoester of formula (VI) is halogenated with sulfuryl chloride or elemental bromine at a temperature between 10°C and the boiling point of the mixture, more preferably with stirring for

a period of from 1 to 30 hours.

Preferably, the halogenated compound prepared from said activated 3-chloro-5-isoxazolepropionic acid or said β-ketoester of formula (VI) is treated with a base which is an alkali metal hydroxide at a temperature of from 30°C to the boiling point of the mixture. The alkali metal hydroxide is preferably sodium hydroxide, and the treatment with an acid is effected by means of a mineral acid (e.g. hydrochloric acid), with cooling.

The process of the invention is illustrated in detail by the following non-limiting Examples.

## EXAMPLE 1

### 3-Chloro-5-isoxazolepropionyl chloride

A mixture containing 17.56 g (0.1 mole) of 3-chloro-5-isoxazolepropionic acid in 20 ml (0.28 mole) of thionyl chloride was stirred overnight. After evaporating the excess thionyl chloride, the residue was subjected to distillation under reduced pressure, using a maximum bath temperature of 160°C, to give the title compound in a yield of 19 g (98.0%), boiling point 90-92°C/0.2 mmHg (27 Pa).

Elemental Analysis:

Calculated for $C_6H_5Cl_2NO_2$ (molecular weight 194.03): C, 37.14%; H, 2.60%; Cl, 36.55%; N, 7.22%.

Found: C, 36.97%; H, 2.72%; Cl, 36.38%; N, 7.17%.

Infrared Absorption Spectrum (liquid film) $\nu_{max} cm^{-1}$:

3100, 1770.

Nuclear Magnetic Resonance Spectrum ($CDCl_3$) $\delta$ ppm:

3.0-3.65 (4H, multiplet);

6.17 (1H, singlet).

## EXAMPLE 2

### (a) Methyl 3-chloro-5-isoxazolepropionate

10 ml of concentrated sulfuric acid were added to a solution of 45 g (0.26 mole) of 3-chloro-5-isoxazolepropionic acid in 300 ml of methanol. The mixture was heated under reflux for 10 hours. Two-thirds of the solvent was then distilled off using a water jet pump and, after cooling, the residue was poured onto 500 g of ice. The resulting product was

filtered off, washed with water, taken up in 300 ml of diethyl ether, washed successively with 60 ml of a 10% aqueous sodium carbonate solution and then with 60 ml of water, dried over anhydrous magnesium sulfate and evaporated, to give 32 g (65%) of the title compound melting at 35.5°C (after recrystallization from hexane).

Elemental Analysis:

Calculated for $C_7H_8ClNO_3$ (molecular weight 189.6):
C, 44.34%; H, 4.25%; N, 7.39%; Cl, 18.70%.

Found: C, 44.36%; H, 4.52%; N, 7.18%; Cl, 18.93%.

Infrared Absorption Spectrum (KBr) $\nu_{max}cm^{-1}$:
3130, 1730, 1200, 1000.

Nuclear Magnetic Resonance Spectrum ($CDCl_3$) $\delta$ ppm:
2.58-3.36 (4H, multiplet);
3.71 (3H, singlet);
6.10 (1H, singlet).

(b) Ethyl 3-chloro-5-isoxazolepropionate

The process described in Example 2(a) above was repeated, but using ethanol, to give 37 g (70%) of the

title compound boiling at 105°C/0.05 mmHg (7 Pa).

Elemental Analysis:

Calculated for $C_8H_{10}ClNO_3$ (molecular weight 203.6):

C, 47.18%; H, 4.95% Cl, 17.41%; N, 6.88%.

Found: C, 47.25%; H, 5.12%; Cl, 17.27%; N, 6.98%.

Infrared Absorption Spectrum (liquid film)

$\nu_{max} cm^{-1}$:

3170, 1740, 1200, 1020.

Nuclear Magnetic Resonance Spectrum ($CDCl_3$) $\delta$ ppm:

1.16 (1H, singlet);

1.26 (5H, t+4, 160, J=7Hz);

2.58-3.36 (4H, multiplet).

<u>(c) Ethyl 5-(3-chloro-5-isoxazolyl)-2-(3-chloro-5-isoxazolylmethyl)-3-oxopentanoate</u>

5.09 g (25 mmoles) of ethyl 3-chloro-5-isoxazolepropionate were dissolved in 20 ml (174 mmoles) of diethyl carbonate in a flask equipped with a Claisen device and a dropping funnel. The reaction mixture was heated to boiling under a pressure of 125 mmHg

($1.7 \times 10^4$ Pa), and a solution of 0.50 g (26 mmoles) of sodium in 13 ml of anhydrous ethanol was added over 15 minutes. The reaction mixture was warmed until the distillation of ethanol ceased (5 to 6 hours). Then, diethyl carbonate was evaporated under reduced pressure, the residue was thoroughly triturated twice, each time with 50 ml of diethyl ether, and the ethereal solution was decanted. The insoluble thick oil was covered with 50 ml of diethyl ether, acidified by adding acetic acid (about 1.5 ml) and stirred until the sodium acetate which formed separated as a white powder. The inorganic material was removed from the suspension by washing twice, each time with 10 ml of water, and then the organic solution was dried over anhydrous magnesium sulfate. After evaporating the ethereal solution, the oily residue weighing 4.3 g was purified by column chromatography on 150 g of Kieselgel 60 ϕ 0.063-02 adsorbent using toluene and then a 10:2 mixture of toluene and acetone as eluent. The title compound was obtained in a yield of 2.5 g (57%).

Elemental Analysis:

Calculated for $C_{14}H_{14}Cl_2N_2O_2$ (molecular weight 361.18):
C, 46.55%; H, 3.90%; Cl, 19.63%; N, 7.76%.

Found: C, 46.36%; H, 4.09%; Cl, 19.79%; N, 8.06%.

Infrared Absorption Spectrum (liquid film)

$\nu_{max} cm^{-1}$:

3130, 1740, 1720, 1600, 1020.

Nuclear Magnetic Resonance Spectrum ($CDCl_3$) $\delta$ ppm:

1.21 (5H, t+4, 26q);

3.09-3.59 (4H, multiplet);

3.38 (2H, doublet);

4.26 (1H, triplet);

6m 15s ls 6m 17s (isoxazole -4H).

EXAMPLE 3

(a) E-3-chloro-5-isoxazoleacrylic acid

A mixture containing 97.0 g (0.5 mole) of 3-chloro-5-isoxazolepropionyl chloride, 100 ml of sulfuryl chloride and 1 g of dibenzoyl peroxide in 400 ml of anhydrous carbon tetrachloride was boiled under reflux for 10 hours, and then the same amount of sulfuryl chloride and dibenzoyl peroxide were added and the mixture was again boiled under reflux for 10 hours. The mixture was evaporated under reduced pressure and the remaining oil was poured into a solution of 80 g (2 moles) of sodium hydroxide in 300 ml of water, whilst cooling with ice-water. After diluting with 600 ml of water, the mixture was heated to boiling, and then

cooled to 30°C, extracted twice, each time with 500 ml of diethyl ether, and the ethereal layer was discarded. The aqueous phase was acidified to pH 2 by adding concentrated hydrochloric acid. The crystalline precipitate was filtered, washed with a little cold water and dried to give 71 g of the title compound melting at 200-201°C.

Elemental Analysis:

Calculated for $C_6H_4ClNO_3$ (molecular weight 173.56):
C, 41.52%; H, 2.32%; Cl, 20.43%; N, 8.07%.

Found: C, 41.39%; H, 2.40%; Cl, 20.56%; N, 8.01%.

Infrared Absorption Spectrum (KBr) $\nu_{max}cm^{-1}$:
3400-2700, 1680, 1560.

Nuclear Magnetic Resonance Spectrum [$(CD_3)_2SO$] δ ppm:

6.35 (1H, doublet, J=18Hz);
7.00 (1H, singlet);
7.25 (1H, doublet, J=18Hz).

(b) E-3-Chloro-5-isoxazoleacrylic acid

47.0 ml (145.7 g, 0.91 moles) of bromine were dropped into 130 g (0.67 moles) of 3-chloro-5-isoxazole-propionyl chloride over 10 hours at 65-70°C, whilst stirring. The reaction mixture was stirred at the same temperature for a further 10 hours. After cooling, the reaction mixture was poured onto 400 g of ice and 650 ml of a 10% sodium hydroxide solution were added, whilst stirring. The resulting mixture was heated to 90°C and stirred in a bath at 90-95°C for 10 minutes. After cooling, the reaction mixture was treated with charcoal, filtered and acidified to pH 1 with concentrated hydrochloric acid, whilst cooling with ice. The precipitated material was recovered by filtering and washing with water. The raw product obtained (114.6 g) was recrystallized from a 2:1 mixture of water and acetic acid. 57 g of the title compound were obtained. All properties of this substance are identical with those of the product prepared as in Example 2.

(c) E-3-Chloro-5-isoxazoleacrylic acid

0.37 ml (7 mmoles) of bromine was dropped into a solution of 915 mg (2.53 mmoles) of ethyl 5-(3-chloro-5-isoxazolyl)-2-(3-chloro-5-isoxazolylmethyl)-3-oxo-pentanoate in 10 ml of anhydrous diethyl ether, whilst

cooling in ice-water and stirring, whereupon the reaction mixture was allowed to warm to room temperature and then the mixture was stirred on a water bath until the starting material disappeared. (This was observed on a thin layer chromatogram using Kieselgel 60 $pF_{254}$ (Merck) as adsorbent, developing with a solvent system containing toluene and acetone in a 10:05 ratio and then by detecting with iodine vapour; under these conditions the Rf value is 0.5).

After evaporating the ether, the residue was boiled with 10 ml of an aqueous 2N sodium hydroxide solution, then filtered off, and the filtrate was acidified to pH 2 by adding concentrated hydrochloric acid whilst cooling by ice-water. The precipitate was filtered off and washed with a little water to give 502 mg (58% yield) of the title compound, melting at 200-201°C. All properties of this substance are identical with those of the product prepared as described in Example 2.

EXAMPLE 4

(a) E-3-(3-Chloro-5-isoxazolyl)acryloyl chloride

A mixture containing 19 g (0.11 mole) of E-3-chloro-5-isoxazoleacrylic acid, 14 ml (0.2 mole) of thionyl chloride, 1 ml of anhydrous pyridine and 75 ml

of anhydrous benzene was stirred in an oil bath at 110°C for 45 minutes, and then evaporated under reduced pressure. After recrystallization of the residue from hexane, the title compound was obtained in a yield of 18 g (85%), melting at 73°C.

Elemental Analysis:

Calculated for $C_6H_3Cl_2NO_2$ (molecular weight 192.0): Cl, 36.93%; N, 7.29%.

Found: Cl, 36.72%; N, 7.45%.

Infrared Absorption Spectrum (KBr) $\nu_{max} cm^{-1}$:

1730, 1540.

Nuclear Magnetic Resonance Spectrum ($CDCl_3$) δ ppm:

6.50 (1H, singlet);

6.69 (1H, doublet, J=16Hz);

7.42 (1H, doublet, J=16Hz).

(b) 3-Chloro-5-isoxazoleacetaldehyde oxime

6.5 g (100 mmoles) of sodium azide were added in little portions to a solution containing 17 g (88.6 mmoles) of E-3-(3-chloro-5-isoxazolyl)acryloyl chloride in 40 ml of anhydrous dimethylformamide at 0°C over 30 minutes whilst stirring. Then, the reaction mixture was

stirred at 0°C for one hour and then poured into a mixture of 100 g of ice and 300 ml of water. The precipitate was filtered, washed twice, each time with 50 ml of cold water, dissolved in 500 ml of diethyl ether, filtered off, and the ethereal solution was dried over anhydrous calcium chloride. The dried solution was evaporated under reduced pressure at a bath temperature of 40°C. The residue was boiled under reflux with 150 ml of anhydrous dioxane for 45 minutes and this mixture was dropped into a hot solution containing 150 ml of water, 150 ml of glacial acetic acid and 150 ml of concentrated sulfuric acid over 5 minutes with constant stirring. The mixture was boiled for 10 minutes, and then cooled. 30 g (0.43 mole) of hydroxylammonium chloride and 90 g (1.1 mole) of sodium acetate were added, and the mixture was stirred at room temperature for 3 hours. The mixture was diluted with 600 ml of water and extracted 3 times, each time with 250 ml of diethyl ether. The combined ethereal solution was washed 3 times, each time with 250 ml of water, dried over anhydrous calcium chloride and clarified with charcoal, which was then filtered off. The filtrate was evaporated under reduced pressure. The residue was thoroughly triturated with 150 ml of pentane, whereupon the pentane was repeatedly evaporated under reduced pressure. The solidified residue was recrystallized from a mixture containing dichloromethane and pentane in

a ratio of 1:4 to give 9.4 g (66%) of the title compound melting at 71-72°C (E/Z mixture).

Elemental Analysis:

Calculated for $C_5H_5ClN_2O_2$ (molecular weight 160.57): Cl, 22.08%; N, 17.45%.

Found: Cl, 22.57%; N, 17.08%.

Infrared Absorption Spectrum (KBr) $\nu_{max}cm^{-1}$:

3600-3000, 1600.

Nuclear Magnetic Resonance Spectrum ($CDCl_3$+$D_2O$) δ ppm:

3.7 (doublet, J=8.35Hz);

3.9 (doublet, J=6.66Hz);

6.15 (1H, singlet);

6.95 (triplet, J=6.66Hz);

7.55 (triplet, J=8.35Hz).

(c) 3-Chloro-5-isoxazoleacetaldehyde oxime

2.34 g (35 mmoles) of sodium azide dissolved in 6 ml of water were added to a solution containing 5.76 g (30 mmoles) of E-3-(3-chloro-5-isoxazolyl)acryloyl chloride in 20 ml of anhydrous dichloromethane, whilst stirring. The reaction mixture was stirred at room temperature for

an additional 20 minutes. The aqueous phase was separated and extracted with 20 ml of dichloromethane, and the combined organic phase was dried over anhydrous magnesium sulfate. The solution was evaporated at room temperature, the residue was added portionwise to 10 ml of concentrated sulfuric acid whilst stirring, and then the reaction mixture was kept at 30 to 40°C for 3 hours. After gas evolution had ceased, the mixture was poured onto 80 g of ice and made alkaline by adding a solution of 16 g (0.4 mole) of sodium hydroxide in 40 ml of water. The organic phase was separated and the aqueous layer was extracted 3 times, each time with 20 ml of dichloromethane. The combined organic solution was dried over anhydrous magnesium sulfate and evaporated. A solution of 2.1 g (30 mmoles) of hydroxylammonium chloride in 20 ml of ethanol and 10 ml of water was added to the residue. The solution was boiled under reflux for one hour, cooled and poured into 300 ml of water. The mixture was extracted 3 times, each time with 100 ml of dichloromethane and the combined organic phase was dried and evaporated. The residue was rubbed with a little pentane tc give 2.52 g (56% yield) of the title compound, the physical characteristics of which are in complete agreement with those of the substance prepared according to Example 4(b).

(d) 3-Chloro-5-isoxazoleacetic acid

7.9 ml (0.1 mole) of thionyl chloride dissolved in 40 ml of anhydrous diethyl ether were dropped into a solution of 5.5 g (40.5 mmoles) of 3-chloro-5-isoxazoleacetaldehyde oxime in 60 ml of anhydrous diethyl ether at 0°C over 5 minutes. The mixture was then allowed to warm to room temperature and stirred for an additional 30 minutes. After evaporating the mixture under reduced pressure, 60 ml of benzene and then 60 ml of pentane were distilled from the residue. The remaining crude nitrile was boiled under reflux in a solution containing 15 ml of acetic acid and 15 ml of 48% hydrobromic acid in an oil bath kept at 140°C for 90 minutes, and then the reaction mixture was poured into a solution of 80 g of water and 40 g of ice. The insoluble solid material was filtered off, the aqueous phase was extracted 3 times, each time with 75 ml of diethyl ether, and, after washing and drying, the combined organic solution was evaporated. The solid residue was recrystallized from a 1:2 mixture of benzene and hexane to give 4.3 g (66% yield) of the title compound melting at 90-92°C.

Elemental Analysis:

Calculated for $C_5H_4ClNO_3$

(molecular weight 161.54):

Cl, 21.95%; N, 8.76%.

Found: Cl, 21.85%; N, 8.90%.

Infrared Absorption Spectrum (KBr) $\nu_{max}cm^{-1}$:

3500-2400, 1700.

Nuclear Magnetic Resonance Spectrum ($CDCl_3$) $\delta$ ppm:

3.9 (2H, singlet);

6.35 (1H, singlet);

10.25 (1H, broad).

CLAIMS:

1. A process for preparing 3-chloro-5-isoxazoleacetic acid by dehydrating 3-chloro-5-isoxazoleacetaldehyde oxime to 3-chloro-5-cyanomethylisoxazole, which is then hydrolyzed to said 3-chloro-5-isoxazoleacetic acid.

2. A process according to claim 1, in which the dehydration is with thionyl chloride.

3. A process according to claim 1 or claim 2, in which the hydrolysis is effected in a strongly acidic medium.

4. A process according to any one of the preceding claims, in which said 3-chloro-5-isoxazoleacetaldehyde oxime is prepared by activating the carboxylic group of E-3-chloro-5-isoxazoleacrylic acid, reacting the activated acid with an azide to give 3-chloro-5-isoxazoleacryloyl azide, subjecting said 3-chloro-5-isoxazoleacryloyl azide to a Curtius degradation, hydrolysing the resulting product with an acid and reacting the hydrolysed product with hydroxylamine.

5. A process according to claim 4, in which said azide is sodium azide.

6. A process according to claim 4 or claim 5, in which the carboxylic acid group of said E-3-chloro-5-isoxazoleacrylic acid is activated by conversion to the acid chloride.

7. A process according to any one of claims 4 to 6, in which said E-3-chloro-5-isoxazoleacrylic acid is prepared by activating the carboxylic acid group of 3-chloro-5-isoxazolepropionic acid, reacting the activated acid with a halogenating agent to halogenate the 5- side chain thereof, and then treating the halogenated compound with a strong base and then with an acid.

8. A process according to claim 7, in which said halogenating agent is sulfuryl chloride or bromine.

9. A process according to claim 7 or claim 8, in which the carboxylic acid group of 3-chloro-5-isoxazolepropionic acid is activated by forming the acid chloride thereof.

10. A process according to any one of claims 4, 5 and 6, in which said E-3-chloro-5-isoxazoleacrylic acid is prepared by reacting an ester of formula (V):

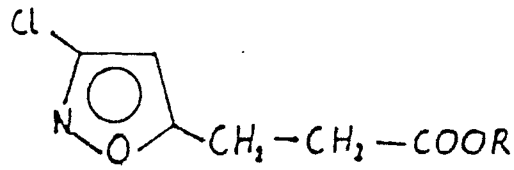

(V)

(in which R represents a $C_1$-$C_6$ alkyl group) with a strong base to prepare a β-ketoester of formula (VI):

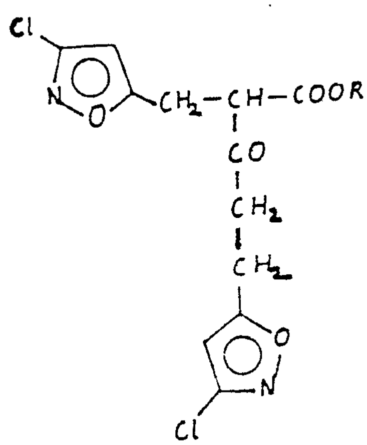

(VI)

(in which R is as defined above), halogenating said β-ketoester and treating the resulting halogenated compound with a base and then with an acid.

11. A process according to claim 10, in which the halogenation is effected by means of bromine or chlorine.

12. A process according to claim 10 or claim 11, in which said strong base is a sodium alkoxide, sodium hydride, sodium amide or potassium t-butoxide.

13. A process according to any one of claims 10 to 12, in which said ester of formula (V) is formed by esterification of 3-chloro-5-isoxazolepropionic acid.

14. A process according to any one of claims 4 to 13 in which the activated 3-chloro-5-isoxazolepropionic acid or said β-ketoester of formula (VI) is halogenated with sulfuryl chloride or elemental bromine at a temperature between 10°C and the boiling point of the mixture.

15. A process according to claim 14, in which the halogenation is effected with stirring for a period of from 1 to 30 hours.

16. A process according to any one of claims 4 to 15, in which the halogenated compound prepared from said activated 3-chloro-5-isoxazolepropionic acid or said β-ketoester of formula (VI) is treated with a base which is an alkali metal hydroxide at a temperature of from 30°C to the boiling point of the mixture.

17. A process according to claim 16, in which said alkali metal hydroxide is sodium hydroxide.

18. A process according to any one of claims 4 to 17 in which the treatment with an acid is effected by means of

a mineral acid, with cooling.

19. A process according to claim 18, in which said mineral acid is hydrochloric acid.

European Patent Office

# EUROPEAN SEARCH REPORT

0219990

Application number

EP 86307432.4

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| D,A | GB - A - 1 464 377 (GIST-BROCADES)<br>* Formula VIII *<br>-- | 1 | C 07 D 261/10 |
| D,A | CHEMICAL ABSTRACTS, vol. 96, no. 7, February 15, 1982, Columbus, Ohio, USA<br>LUGOSI, PETER; SCHAWARTZ, JOZSEF; DOLESCHALL, GABOR. "Synthesis of 3-haloisoxazoles by novel oxidative degradation of the side-chain of 3-(3-haloisoxazol-5-yl)propionic acids." page 612, column 2, abstract no. 52209b<br>& Tetrahedron 1981, 37(17), 3061-5 (Eng.).<br>-- | 1 | |
| A | HOUBEN-WEYL "Methoden der organischen Chemie", 4th edition, vol. E5: "Carbonsäuren und Carbonsäure-Derivate", 1985<br>GEORG THIEME VERLAG, Stuttgart-New York pages 265-268, 1339<br>---- | 1 | TECHNICAL FIELDS SEARCHED (Int. Cl.4)<br>C 07 D 261/OC |

The present search report has been drawn up for all claims

| Place of search | Date of completion of the search | Examiner |
|---|---|---|
| VIENNA | 17-12-1986 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82